(19) Europäisches Patentamt
European Patent Office
Office européen des brevets

(11) Veröffentlichungsnummer: **0 507 238 A1**

(12) # EUROPÄISCHE PATENTANMELDUNG

(21) Anmeldenummer: **92105476.3**

(51) Int. Cl.[5]: **C07C 59/68**

(22) Anmeldetag: **30.03.92**

(30) Priorität: **05.04.91 DE 4111026**

(43) Veröffentlichungstag der Anmeldung:
**07.10.92 Patentblatt 92/41**

(84) Benannte Vertragsstaaten:
**PT**

(71) Anmelder: **BOEHRINGER MANNHEIM GMBH**
**Sandhofer Strasse 116**
**W-6800 Mannheim 31(DE)**

(72) Erfinder: **Wolff, Hans Peter, Dr.**
**Untere Clignet Strasse 4**
**W-6800 Mannheim 1(DE)**
Erfinder: **Kuehnle, Hans-Frieder, Dr.**
**Silcher Weg 6**
**W-6940 Weinheim(DE)**

(54) **Optisch aktive Carbonsäuren sowie diese enthaltende Arzneimittel.**

(57) Gegenstand der vorliegenden Erfindung sind R- und S-Carbonsäuren der Formel I

$$\begin{array}{l} \qquad\quad\;\; * \\ R_1\text{-}A\text{-}CH\text{-}COOH \\ \qquad\quad\;\; | \\ \qquad\quad\;\; Y\text{-}B\text{-}R_2 \end{array} \qquad (I),$$

in welcher

- $R_1$ einen Aryl-, Aryloxy-, Arylthio-, Arylsulfinyl-, Arylsulfonyl- oder Arylaminorest, wobei die Arylteile jeweils durch einen oder mehrere Reste aus der Gruppe Hydroxy, Halogen, $C_1$-$C_8$-Alkyl, $C_1$-$C_8$-Alkoxy, Trifluormethyl, Cyano, Nitro, Amino, $C_1$-$C_8$-Alkylamino oder Di-$C_1$-$C_8$-Alkylamino substituiert sein können,
- $R_2$ einen Arylrest, der durch einen oder mehrere Reste aus der Gruppe Hydroxy, Halogen, $C_1$-$C_8$-Alkyl, $C_1$-$C_8$- Alkoxy, Trifluormethyl, Cyano, Nitro, Amino, $C_1$-$C_8$-Alkylamino oder Di-$C_1$-$C_8$-Alkylamino substituiert sein kann,
- A einen geradkettigen oder verzeigten, gesättigten oder ungesättigten aliphatischen Rest mit 3-10 Kohlenstoffatomen, der gegebenenfalls durch ein Heteroatom unterbrochen ist, mit der Maßgabe, daß an einem ungesättigten aliphatischen Kohlenstoffatom kein Heteroatom stehen soll,
- Y die Gruppen $-S(O)_n-$ oder -O-,
- n die Zahlen 0, 1 oder 2 und
- B einen Valenzstrich oder einen geradkettigen oder verzweigten, gesättigten oder ungesättigten aliphatischen Rest mit 1 bis 5 Kohlenstoffatomen bedeutet,

sowie deren physiologisch unbedenklichen Salze und Ester.

Die vorliegende Erfindung betrifft ferner Arzneimittel, die Verbindungen der Formel I enthalten, zur Behandlung von Diabetes, Prädiabetes und insbesondere zur Behandlung von Altersdiabetes. Zusätzlich zeigen die erfindungsgemäßen Substanzen eine ausgeprägte lipidsenkende Wirkung und eignen sich daher auch zur Behandlung von Fettstoffwechselerkrankungen.

EP 0 507 238 A1

Gegenstand der vorliegenden Erfindung sind R- und S-Carbonsäuren der Formel I

$$\begin{array}{l} \qquad\quad * \\ R_1\text{-}A\text{-}CH\text{-}COOH \\ \qquad\quad | \\ \qquad\quad Y\text{-}B\text{-}R_2 \end{array} \qquad (I),$$

in welcher

- $R_1$ einen Aryl-, Aryloxy-, Arylthio-, Arylsulfinyl-, Arylsulfonyl- oder Arylaminorest, wobei die Arylteile jeweils durch einen oder mehrere Reste aus der Gruppe Hydroxy, Halogen, $C_1$-$C_8$-Alkyl, $C_1$-$C_8$-Alkoxy, Trifluormethyl, Cyano, Nitro, Amino, $C_1$-$C_8$-Alkylamino oder Di-$C_1$-$C_8$-Alkylamino substituiert sein können,
- $R_2$ einen Arylrest, der durch einen oder mehrere Reste aus der Gruppe Hydroxy, Halogen, $C_1$-$C_8$-Alkyl, $C_1$-$C_8$- Alkoxy, Trifluormethyl, Cyano, Nitro, Amino, $C_1$-$C_8$-Alkylamino oder Di-$C_1$-$C_8$-Alkylamino substituiert sein kann,
- A einen geradkettigen oder verzeigten, gesättigten oder ungesättigten aliphatischen Rest mit 3-10 Kohlenstoffatomen, der gegebenenfalls durch ein Heteroatom unterbrochen ist, mit der Maßgabe, daß an einem ungesättigten aliphatischen Kohlenstoffatom kein Heteroatom stehen soll,
- Y die Gruppen $-S(O)_n-$ oder -O-,
- n die Zahlen 0, 1 oder 2 und
- B einen Valenzstrich oder einen geradkettigen oder verzweigten, gesättigten oder ungesättigten aliphatischen Rest mit 1 bis 5 Kohlenstoffatomen bedeutet,

sowie deren physiologisch unbedenklichen Salze und Ester.

Die vorliegende Erfindung betrifft ferner Arzneimittel, die Verbindungen der Formel I enthalten, zur Behandlung von Diabetes, Prädiabetes und insbesondere zur Behandlung von Altersdiabetes. Zusätzlich zeigen die erfindungsgemäßen Substanzen eine ausgeprägte lipidsenkende Wirkung und eignen sich daher auch zur Behandlung von Fettstoffwechselerkrankungen.

In den Patentanmeldungen WO-A-87/00521 und EP-A-0,279,162 sind Carbonsäuren der Formel I beschrieben, wobei sich die in diesen Anmeldungen enthaltenen Ausführungsbeispiele ausschließlich auf die Herstellung von Racematen beziehen. Die dort beschriebenen Verbindungen besitzen als gemeinsames Strukturelement einen Substituenten in alpha-Stellung zur Carboxylfunktion. Diese Carbonsäuren weisen wertvolle pharmakologische Eigenschaften auf. Insbesondere sind sie geeignet, eine verminderte Insulinsensitivität, wie sie bei diabetischer Stoffwechsellage auftritt, wieder zu normalisieren.

Überraschend wurde nun gefunden, daß bei diesen Substanzen, die bisher nur als Racemate bekannt waren, die Wirkung hauptsächlich einem der Enantiomeren, vorzugsweise der linksdrehenden Form, zugeschrieben werden kann. Durch Auswahl geeigneter optisch reiner R- oder S-Isomere gelingt es, die gewünschte pharmakologische Wirkung bereits bei einer Dosis zu erreichen, die sich gegenüber der weniger wirksamen Form um den Faktor 4-100 verringert hat. Dies ist insbesondere deshalb von großem therapeutischen Nutzen, da bei der Verabreichung des pharmakologisch aktiven Isomers anstelle des Racemats die eventuell auftretenden Nebenwirkungen entfallen, die der pharmakologisch unwirksamen Form zugeschrieben werden können. Somit kann eine deutliche Erhöhung der therapeutischen Breite erreicht werden. Dies hat den Vorteil, daß die pharmakologisch aktiven Isomere in deutlich geringeren Dosen im Vergleich zu den Racematen (Faktor 4-50, in einigen Fällen bis zu Faktor 100) appliziert werden können.

Die erfindungsgemäßen Verbindungen eignen sich insbesondere zur Herstellung von Antidiabetica zur oralen Behandlung des Diabetes mellitus, vor allem des Typs II bzw. Typ-IIb. Damit ist es erstmals gelungen, die periphere Insulinresistenz beim Typ-II-Diabetiker zu beeinflussen. Hierbei spielt nach derzeitigen Erkenntnissen die Verwertungsstörung von Insulin und Glucose als eine der Hauptursachen des Altersdiabetes eine große Rolle. Durch diese Verwertungsstörung wird eine Hyperinsulinämie hervorgerufen, die wiederum als Risikofaktor für die Entstehung makroangiopathischer Komplikationen gilt. Untersuchungen mit adipösen Typ-II-Diabetikern zeigten, daß sich mit den erfindungsgemäßen Substanzen sowohl die Glucose- als auch die Insulinspiegel senken ließen. Aufgrund ihres besonderen Wirkmechanismus haben die Substanzen weiterhin einige Vorteile: Sie verursachen keine Hypoglykämien und können, da sie auch den Insulinspiegel reduzieren, das Arterioskleroserisiko des Typ-II-Diabetikers senken. Sie eignen sich daher auch zur Prophylaxe vor aterosklerotischen Erkrankungen. Außerdem besitzen sie einen positiven

Einfluß auf erhöhte Blutdruckwerte und bewirken eine Senkung der Triglycerid- und Cholesterinspiegel.

Unter Arylresten werden in allen Definitionen aromatische Kohlenstoffwasserstoffe mit 6 bis 14 Kohlenstoffatome verstanden, bevorzugt die Phenyl- und Naphthylgruppe.

Unter substituierten Arylresten werden in allen Definitionen solche aromatische Kohlenstoffe mit 6 bis 14 Kohlenstoffatomen verstanden, die in einer oder mehreren Positionen eine Hydroxyl, Halogen-, $C_1$-$C_8$-Alkyl-, $C_1$-$C_8$-Alkoxy-, Trifluormethyl-, Cyano-, Nitro- oder gegebenenfalls ein- oder zweifach durch $C_1$-$C_8$-Alkyl substituierte Aminogruppe tragen. Bevorzugt tragen die jeweiligen "Aryl"-teile ein, zwei oder drei der oben genannten Substituenten. Bevorzugt kommen solche "Alkyl"-teile in Frage, die 1-6, insbesondere 1-4 C-Atome besitzen, wie z.B. Methyl, Ethyl, n-Propyl, i-Butyl, t-Butyl oder Neopentyl. Besonders bevorzugte Arylteile sind der Phenyl-, 4-Methylphenyl-, 4-t-Butylphenyl-, 4-Methoxyphenyl-, 2-Methoxyphenyl-, 3-Trifluormethylphenyl- und der 4-Chlorphenylrest.

Unter Halogen versteht man Fluor, Chlor, Brom und Jod, vorzugsweise Fluor, Chlor und Brom.

Für unverzweigte aliphatische Gruppen A kommen insbesondere die folgenden Alkylengruppen bzw. die durch ein Heteroatom X unterbrochenen Alkylengruppen in Frage:

a) $-(CH_2)_o-$ mit o = 3-10 bzw.
$-(CH_2)_p-X-(CH_2)_q-$ mit p = 2.8 und q = 1-6,
wobei o, p und q ganze Zahlen bedeuten, die Summe von p und q nicht größer als 10 sein darf und X ein Sauerstoff- oder Schwefelatom oder die Gruppe NH bedeutet.

b) $-CH_2-X-(CH_2)_q-$, $-CH=CH-CH_2-$, $-C\equiv C-CH_2-$, $-C\equiv C(CH_2)_p-$,
worin p, q und X die oben angegebenen Bedeutungen besitzen. Bevorzugt bedeutet $R_1$ dann einen Arylrest.

Als verzweigte aliphatische Reste kommen zum Beispiel in Betracht $-CH_2-CH(CH_3)-CH_2-$ und $-CH=C(CH_3)-CH_2-$, wobei $R_1$ dann bevorzugt einen Arylrest bedeutet.

Unter einer Alkylengruppe B sind insbesondere die Reste $-(CH_2)_r-$, $-CH_2-CH=CH-$, $-CH_2-C\equiv C-$ und $-CH=CH-$ zu verstehen, worin r eine ganze Zahl von 1 bis 5 bedeutet.

Die Verbindungen der Formel I können als freie Säure oder in Form der physiologisch unbedenklichen Salze mit starken oder schwachen Basen, wie z.B. Natronlauge, Kalilauge oder Ammoniak, vorliegen. Als physiologisch unbedenkliche Salze kommen insbesondere Alkali-, Erdalkali- oder Ammoniumsalze (sowie gegebenenfalls Salze mit blutzuckersenkenden Biguaniden) in Frage. Die Carboxylgruppe kann auch mit niederen Alkoholen, wie z.B. Methanol, Ethanol, Propanol, in die entsprechenden physiologisch verträglichen Ester umgewandelt werden. Die von den Carbonsäuren der allgemeinen Formel I abgeleiteten Ester enthalten als Alkoholkomponente niedere einwertige Alkohole mit 1-6 C-Atomen, von denen Methanol, Ethanol und n-Butanol bevorzugt sind, sowie mehrwertige Alkohole mit 2-6 C-Atomen, wie z.B. Glycerin, oder Alkohole mit anderen funktionellen Gruppen, wie z.B. Ethanolamin.

Gegenstand der Erfindung sind weiterhin Verfahren zur Herstellung der R- und S-Isomere von optisch aktiven Verbindungen der allgemeinen Formel I, indem man

a) in an sich bekannter Weise ein racemisches Gemisch von Verbindungen der allgemeinen Formel I mit optisch aktiven Basen, wie z. B. Ephedrin, umsetzt und die entstandenen diastereomeren Salze durch physikalische Methoden, wie z.B. fraktionierte Kristallisation oder Fest-Flüssigchromatographie, trennt und die Säuren wieder freisetzt,

b) in an sich bekannter Weise ein racemisches Gemisch eines niederen Esters von Verbindungen der allgemeinen Formel I mit einem Enzym wie Esterase oder Lipase enantioselektiv spaltet oder

c) in an sich bekannter Weise eine optisch aktive Verbindung der allgemeinen Formel II

$$R_1-A-\overset{*}{C}H(X)-COOR_3 \qquad (II),$$

S-Isomer oder R-Isomer

in welcher $R_1$ und A die oben angegebenen Bezeichnungen besitzen und X eine Abgangsgruppe wie z.B. Halogen oder einen Sulfonsäureester, wie z.B. die Trifluormethylsulfonyloxy- oder 4-Chlorphenylsulfonyloxygruppe, und $R_3$ einen $C_1$-$C_8$-Alkylrest bedeuten,
mit einer Verbindung der allgemeinen Formel III

$H$-$Y$-$B$-$R_2$ (III),

in welcher B und $R_2$ die oben angegebenen Bedeutungen besitzen und Y S oder O bedeutet, umsetzt und die erhaltenen Ester der Verbindungen der allgemeinen Formel I im Anschluß daran durch Verseifen in die freien Carbonsäuren der allgemeinen Formel I überführt und gegebenenfalls die Derivate mit Y = S in an sich bekannter Weise durch Oxidation des Schwefels in Derivate mit Y = SO bzw. $SO_2$ überführt.

Optisch aktive Verbindungen der allgemeinen Formel II können hergestellt werden, indem man

a) optisch aktive Verbindungen der allgemeinen Formel IV

$$R_1\text{-}A\text{-}\overset{*}{C}H(OH)\text{-}COOR_3 \qquad \text{(IV)}$$

R-Isomer oder
S-Isomer

in welcher $R_1$, A und $R_3$ die oben angegebenen Bedeutungen haben, halogeniert oder sulfoniert, oder

b) racemische Gemische von Verbindungen der allgemeinen Formel II, in denen $R_3$ Wasserstoff bedeutet und $R_1$ und A die oben angegebenen Bedeutungen besitzen, über diastereomere Salze mit optisch aktiven Basen, wie z. B. Ephedrin, in an sich bekannter Weise trennt, die Säuren wieder freisetzt und durch Verestern in die optisch aktiven Ester der allgemeinen Formel II überführt.

Optisch aktive Verbindungen der allgemeinen Formel IV sind erhältlich durch

a) stereoselektive Reduktion von Verbindungen der allgemeinen Formel V nach an sich bekannten Verfahren (H.C. Brown, G.G. Pai und P.K. Jadhav, J.Am.Chem.Soc. 1984, (106), 1531)

$R_1$-A-CO-$COOR_3$ (V),

in welcher $R_1$, A und $R_3$ die oben angegebenen Bedeutungen besitzen oder

b) durch Trennung racemischer Gemische von Verbindungen der allg. Formel IV, in denen $R_3$ Wasserstoff bedeutet und $R_1$ und A die oben angegebenen Bedeutungen besitzen, über diastereomere Salze mit optisch aktiven Basen und anschließende Freisetzung und Überführung der optisch aktiven Säuren der allgemeinen Formel IV in die gewünschten Ester mit den oben angegebenen Bedeutungen für $R_3$.

Die Herstellung des racemischen Gemisches von Verbindungen der allgemeinen Formel I ist in den Patentanmeldungen WO-A-87/00521 und EP-A-0,279,162 beschrieben.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Bevorzugt im Sinne der Erfindung sind außer den in den Beispielen genannten die R- und S-Isomere von folgenden Verbindungen der Formel I sowie deren Salze und Ester, insbesondere die linksdrehenden (-)-Isomere.

1. 5-(4-Chlorphenyl)-2-[4-(1.1-dimethylethyl)phenoxy]-pentansäure
2. 2-(4-Methylphenoxy)-4-(2-phenylethoxy)buttersäure
3. 4-[2-(4-Chlorphenyl)ethoxy]-2-[4-(1.1-dimethylethyl)phenoxy]buttersäure
4. 4-[2-(4-Chlorphenyl)ethylamino]-2-[4-(1.1-dimethylethyl)phenoxy]buttersäure
5. 5-(4-Chlorphenylmethoxy)-2-(4-methylphenoxy)pentansäure
6. 2-[4-(1.1-dimethylethyl)phenoxy]-6-phenoxyhexansäure
7. 6-(2-Chlorphenoxy)-2-(4-methylphenoxy)hexansäure
8. 6-(4-Chlorphenoxy)-2-(4-cyanophenoxy)hexansäure
9. 6-(4-Chlorphenoxy)-2-(4-dimethylaminophenoxy)hexansäure
10. 6-(4-Fluorphenoxy)-2-(4-methylphenoxy)hexansäure
11. 6-(4-Hydroxyphenoxy)-2-(4-methylphenoxy)hexansäure
12. 2-[4-(1.1-Dimethylethyl)phenylthio]-6-phenoxyhexansäure
13. 6-(4-Chlorphenylthio)-2-(4-methylphenoxy)hexansäure
14. 6-(4-Chlorphenylsulfonyl)-2-(4-methylphenoxy)hexansäure
15. 6-(4-Chlorphenylsulfonyl)-2-[4-(1.1-dimethylethyl)phenoxy]-hexansäure
16. 6-(4-Chlorphenylsulfonamido)-2-[4-(1.1-dimethylethyl)phenoxy]-hexansäure
17. 6-(4-Chlorphenylamino)-2-[4-(1.1-dimethylethyl)phenoxy]-hexansäure
18. 2-[4-(1.1-Dimethylethyl)phenoxy]-7-phenylheptansäure
19. 2-[4-(1.1-Dimethylethyl)phenoxy]-7-(4-methylphenyl)-heptansäure
20. 2-[4-(1.1-Dimethylethyl)phenoxy]-7-[4-(1.1-dimethylethyl)phenyl]-heptansäure
21. 7-(4-Chlorphenyl)-2-(4-butylphenoxy)heptansäure
22. 2-[3.5-Bis-(1.1-dimethylethyl)phenoxy]-7-(4-chlorphenyl)heptansäure
23. 7-(4-Chlorphenyl)-2-[4-(1.1.3.3-tetramethylbutyl)phenoxy]heptansäure
24. 2-(4-Chlorphenoxy)-7-(4-chlorphenyl)heptansäure
25. 7-(4-Chlorphenyl)-2-(3.5-dimethyl-4-hydroxyphenoxy)heptansäure
26. 2-[3.5-Bis-(1.1-dimethylethyl)-4-hydroxyphenoxy]-7-(4-chlorphenyl)heptansäure
27. 7-(4-Chlorphenyl)-2-(4-methoxyphenoxy)heptansäure
28. 7-(4-Chlorphenyl)-2-[4-(1.1-dimethylethoxy)phenoxy]heptansäure
29. 7-(4-Chlorphenyl)-2-(3-trifluormethylphenoxy)heptansäure
30. 7-(3.5-Dimethyl-4-hydroxyphenyl)-2-(4-methylphenoxy)heptansäure
31. 2-[4-(1.1-Dimethylethyl)phenoxy]-7-(3.5-dimethyl-4-hydroxyphenyl)-heptansäure
32. 7-[3.5-Bis-(1.1-dimethylethyl)-4-hydroxyphenyl]-2-[4-(1.1-dimethylethyl)phenoxy]heptansäure
33. 2-[4-(1.1-Dimethylethyl)phenoxy]-7-(3-trifluormethylphenyl)heptansäure
34. 7-(2-Dimethylaminophenyl)-2-[4-(1.1-dimethylethyl)phenoxy]heptansäure
35. 2-[4-(1.1-Dimethylethyl)phenylthio]-7-phenylheptansäure
36. 7-(4-Chlorphenyl)-2-[4-(1.1-dimethylethyl)phenylthio]heptansäure
37. 7-(4-Chlorphenyl)-2-[2-(4-methylphenyl)ethylthio]heptansäure
38. 2-[4-(1.1-dimethylethyl)phenoxy]-8-phenyloctansäure
39. 8-(4-Chlorphenyl)-2-(4-methylphenoxy)octansäure
40. 8-(4-Chlorphenoxy)-2-(4-methylphenoxy)octansäure

## Beispiel 1

### (+)-7-(4-Chlorphenyl)-2-[4-(1,1-dimethylethyl)phenoxy]heptansäure-Natriumsalz

Eine Lösung von 145 g (0.373 mol) racemischer 7-(4-Chlorphenyl)-2-[4-(1,1-dimethylethyl)phenoxy]-heptansäure in einem Gemisch von 50 ml Essigsäureethylester und 150 ml Isohexan wird mit einer Lösung von 61.6 g (0.373 mol) (1R,2S)-(-)-Ephedrin in einem Gemisch von 50 ml Essigsäureethylester und 150 ml Isohexan versetzt. Es kristallisiert langsam ein Niederschlag, den man 3 mal aus Essigester umkristallisiert. Man erhält 100.9 g (49 %, bezogen auf eingesetztes Racemat) (-)-7-(4-Chlorphenyl)-2-[4-(1,1-dimethylethyl)-phenoxy]heptansäure-[(1R,2S)-(-)-Ephedrin]salz, D: -6.9° (c = 1 %, Methanol).

100 g (0.18 mol) (-)-7-(4-Chlorphenyl)-2-[4-(1,1-dimethylethyl)phenoxy]heptansäure-(1R,2S)-(-)-Ephedrin]salz werden unter Eiskühlung und Rühren in 250 ml 2 N Salzsäure eingetragen und das Gemisch mit Ether extrahiert. Die Extrakte werden getrocknet und eingedampft. Man erhält 70.2 g (quant. Ausb.) (-)-7-(4-Chlorphenyl)-2-[4-(1,1-dimethylethyl)phenoxy]-heptansäure, farbloses Öl, D: + 13.5° (c = 1 %, Methanol).

Ein Gemisch von 70 g (0.18 mol) (-)-7-(4-Chlorphenyl)-2-[4-(1,1-dimethylethyl)phenoxy]heptansäure, 200 ml Ethanol und 70 ml Wasser wird unter Rühren tropfenweise mit 90 ml 2 N Natronlauge versetzt. Man rührt, bis eine klare Lösung entstanden ist und dampft das Ethanol ab.

Der Rückstand wird mit Wasser verdünnt, mit Kohle geklärt und zur Trockne eingedampft.
Ausbeute: 68.6 g (93 % d.Th.) (+)-7-(4-Chlorphenyl)-2-[4-(1,1-dimethylethyl)phenoxy]heptansäure-Natriumsalz, amorphes Pulver, D: +11.9° (c = 1 %, Methanol).

In Analogie zu Beispiel 1 erhält man aus (1R,2S)-(-)-Ephedrin und

a) rac. 7-(2-Methoxyphenyl)-2-(4-methylphenoxy)heptansäure
(+)-7-(2-Methoxyphenyl)-2-(4-methylphenoxy)heptansäure-Natriumsalz, Fp. >270° C (amorph).

b) rac. 6-Phenoxy-2-[4-(1.1-dimethylethyl)phenoxy]heptansäure
(+)-6-Phenoxy-2-[4-(1.1-dimethylethyl)phenoxy]heptansäure-Natriumsalz, Fp. >300° C.

c) rac. 7-(2-Methoxyphenyl)-2-[4-(1.1-dimethylethyl)phenoxy]-heptansäure
(+)-7-(2-Methoxyphenyl)-2-[4-(1.1-dimethylethyl)phenoxy]-heptansäure-Natriumsalz, Fp. >270° C, D: +6.7° (c = 1 %, Methanol).

d) rac. 7-(2-Methoxyphenyl)-2-[4-(1-methylethyl)phenoxy]-heptansäure
(+)-7-(2-Methoxyphenyl)-2-[4-(1-methylethyl)phenoxy]-heptansäure-Natriumsalz.

e) rac. 5-(4-Chlorphenyl)-2-[4-1(1.1-dimethyl-ethyl)phenoxy]pentansäure
(+)-5-(4-chlorphenyl)-2-[4-(1.1-dimethylethyl)phenoxy]-pentansäure-Natriumsalz.

## Beispiel 2

(-)-7-(4-Chlorphenyl)-2-[4-(1,1-dimethylethyl)phenoxy] heptansäure-Natriumsalz

Eine Lösung von 145 g (0.373 mol) racemischer 7-(4-Chlorphenyl)-2-[4-(1,1-dimethylethyl)phenoxy]-heptansäure in einem Gemisch von 50 ml Essigsäureethylester und 150 ml Isohexan wird mit einer Lösung von 65 g (0.373 mol) (1S,2R)-(+)-Ephedrin Hemihydrat in einem Gemisch aus 50 ml Essigsäureethylester und 150 ml Isohexan versetzt. Die entstandenen Kristalle werden abgesaugt und 3 mal aus Essigsäureethylester umkristallisiert. Man erhält 99.8 g (48 %, bezogen auf eingesetztes Racemat) (-)-7-(4-Chlorphenyl)-2-[4-(1,1-dimethylethyl)phenoxy]heptansäure-[(1S,2R)-(+)-Ephedrin]salz, D: +6.8° (c = 1 %, Methanol).

99.5 g (0.18 mol) (-)-7-(4-Chlorphenyl)-2-[4-(1,1-dimethylethyl)phenoxy]heptansäure-[(1S,2R)-(+)-Ephedrin]salz werden in Analogie zu Beispiel 1 mit 2 N Salzsäure in die freie Säure überführt. Ausbeute: 69.8 g (quant. Ausb.) (-)-7-(4- Chlorphenyl)-2-[4-(1,1-dimethylethyl)phenoxy] heptansäure, farbloses Öl, D: -13.7° (c = 1 %), Methanol).

69.8 g (0.18 mol) (-)-7-(4-Chlorphenyl)-2-[4-(1,1-dimethylethyl)phenoxy]heptansäure werden in Analogie zu Beispiel 1 mit 2 N Natronlauge in das Natriumsalz überführt. Ausbeute: 69.9 g (95 % d.Th.) (-)-7-(4-Chlorphenyl)-2-[4-(1,1-dimethylethyl)phenoxy]heptansäure-Natriumsalz, amorphes Pulver, D: - 11.3° (c = 1 %, Methanol).

In Analogie zu Beispiel 2 erhält man aus (1S,2R)-(+)-Ephedrin und

a) rac. 7-(2-Methoxyphenyl)-2-(4-methylphenoxy)heptansäure
(-)-7-(2-Methoxyphenyl)-2-(4-methylphenoxy)heptansäure-Natriumsalz, Fp. 290-292° C, D: -6.5° (c= 1 %, Methanol)

b) rac. 6-Phenoxy-2-[4-(1.1-dimethylethyl)phenoxy]heptansäure
(-)-6-Phenoxy-2-[4-(1.1-dimethylethyl)phenoxy]heptansäure-Natriumsalz, Fp. >300° C, D: -2.6° (c = 1 %, Methanol)

c) rac. 7-(2-Methoxyphenyl)-2-[4-(1.1-dimethylethyl)phenoxy]heptansäure
(-)-7-(2-Methoxyphenyl)-2-[4-(1.1-dimethylethyl)phenoxy]heptansäure-Natriumsalz, Fp. >270° C, D: -7.5° - (c = 1 %, Methanol)

d) rac. 7-(2-Methoxyphenyl)-2-[4-(1-methylethyl)phenoxy]heptansäure
(-)-7-(2-Methoxyphenyl)-2-[4-(1-methylethyl)phenoxy]heptansäure-Natriumsalz.

e) rac. 5-(4-Chlorphenyl)-2-[4-(1.1-dimethylethyl)phenoxy]-pentansäure
(-)-5-(4-Chlorphenyl)-2-[4-(1.1-dimethylethyl)phenoxy]-pentansäure-Natriumsalz.

## Beispiel 3

(+)-6-(4-Chlorphenoxy)-2-(4-methylphenoxy)hexansäure

Man versetzt eine Lösung von 28.5 g (82 mmol) racemischer 6-(4-Chlorphenoxy)-2-(4-methylphenoxy)-

hexansäure in 165 ml Essigsäureethylether mit einer Lösung von 13.5 g (82 mmol) (1R,2S)-(-)-Ephedrin in 165 ml Essigsäureethylester. Nach Animpfen scheiden sich langsam Kristalle ab, die abgesaugt und 3 mal aus Essigsäureethylester umkristallisiert werden. Diese Kristalle trägt man unter Rühren und Eiskühlung in 100 ml 1 N Salzsäure ein und extrahiert das Gemisch mit Essigsäureethylester. Die Extrakte werden getrocknet und eingedampft. Ausbeute: 12.6 g (44 %, bezogen auf eingesetztes Racemat) (+)-6-(4-Chlorphenyl)-2-(4-methylphenoxy)hexansäure, Fp. 72-73 °C, D: +5.2° (c = 1 %, Methanol).

In analoger Weise erhält man aus

a) rac. 7-(4-Chlorphenyl)-2-(4-methylphenoxy)heptansäure und (1R,2S)-(-)-Ephedrin (+)-7-(4-Chlorphenyl)-2-(4-methylphenoxy)heptansäure, Fp. 80 °C, D: +13.0° (c = 1 %, Methanol)

b) rac. 7-(4-Chlorphenyl)-2-(4-methylphenylthio)heptansäure und (1R,2S)-(-)-Ephedrin (+)-7-(4-Chlorphenyl)-2-(4-methylphenylthio)heptansäure,Fp. 58-60 °C, D: +62.1° (c = 1 %, Methanol).

c) rac. 5-(4-Chlorphenyl)-2-(4-methylphenylsulfonyl)-4-pentinsäure und (1R,2S)-(-)-Ephedrin (+)-5-(4-Chlorphenyl)-2-(4-methylphenylsulfonyl)-4-pentinsäure, Fp. 133-136 °C, D: +21.3 °C (c = 1 %, Methanol).

## Beispiel 4

### (-)-6-(4-Chlorphenoxy)-2-(4-methylphenoxy)hexansäure

Man versetzt eine Lösung von 24.0 g (69 mmol) racemischer 6-(4-Chlorphenoxy)-2-(4-methylphenoxy)-hexansäure in 140 ml Essigsäureethylester mit einer Lösung von 11.4 g (69 mmol) (1S,2R)-(+)-Ephedrin in 140 ml Essigsäureethylester. Nach Animpfen scheiden sich langsam Kristalle ab, die abgesaugt und 3 mal aus Essigsäureethylester umkristallisiert werden.

Diese Kristalle trägt man unter Rühren und Eiskühlung in 100 ml 1 N Salzsäure ein und extrahiert das Gemisch mit Essigsäureethylester. Die Extrakte werden getrocknet und eingedampft. Den Rückstand verreibt man unter Isohexan. Ausbeute: 11.1 g (46 %, bezogen auf eingesetztes Racemat) (-)-6-(4-Chlorphenyl)-2-(4-methylphenoxy)hexansäure, Fp. 72-74 °C, D: -5.2° (c = 1 %, Methanol).

In analoger Weise erhält man aus

a) rac. 7-(4-Chlorphenyl)-2-(4-methylphenoxy)heptansäure und (1S,2R)-(+)-Ephedrin (-)-7-(4-Chlorphenyl)-2-(4-methylphenoxy)heptansäure, Fp. 79.5 °C, D: -13.1° (c = 1 %, Methanol).

b) rac. 7-(4-Chlorphenyl)-2-(4-methylphenylthio)heptansäure und (1S,2R)-(+)-Ephedrin (-)-7-(4-Chlorphenyl)-2-(4-methylphenylthio)heptansäure,Fp. 59-61° (c = 1 %, Methanol).

c) rac. 5-(4-Chlorphenyl)-2-(4-methylphenylsulfonyl)-4-pentinsäure und (1S,2R)-(+)-Ephedrin (-)-5-(4-Chlorphenyl)-2-(4-methylphenylsulfonyl)-4-pentinsäure, Fp. 134-136 °C, D: -21.8° (c = 1 %, Methanol).

## Beispiel 5

Nachfolgend werden Ergebnisse von pharmakologischen Tests beschrieben, welche beispielhaft die unterschiedliche Wirksamkeit der erfindungsgemäßen Enantiomerenpaare dokumentieren.

Methode:

Die blutglukosesenkende Wirkung der Substanzen wurde an ob/ob-Mäusen mit hereditär-übergewichtigem Typ-II-Diabetes und bestehender Insulin-Resistenz bestimmt. Die Testsubstanzen wurden über 5 Tage hinweg als Suspension in einer Tylose-Lösung an gefütterte Tiere (n = 10) verabreicht. Am fünften Tag wurden die Tiere getötet und aus dem gewonnenen Blut die Blutglukose-Konzentration bestimmt.

Die Ergebnisse in Tabelle 1 sind als prozentuale Senkung gegenüber einer mitgeführten Kontrollgruppe (n = 10) aufgeführt.

Tabelle 1

| Blutglucosesenkung | | | |
|---|---|---|---|
| Verbindung (Bsp.Nr.) | Dosis (mg/kg) | Blutglukose-Senkung (%) | Signifikanz |
| 1 | 30 | 17 | n.s. |
| 2 | 30 | 89 | p<0.01 |
| 3 | 25 | 3 | n.s. |
| 4 | 25 | 51 | p<0.01 |
| 3a | 100 | 31 | n.s. |
| 4a | 100 | 88 | p<0.01 |
| 2a | 25 | 113 | p<0.01 |
| 2c | 25 | 91 | p<0.01 |
| n.s. = nicht signifikant, da p>0.05 | | | |

**Patentansprüche**

**1.** R- und S-Carbonsäuren der allgemeinen Formel I

$$\begin{array}{l} \quad\quad\quad * \\ R_1-A-CH-COOH \\ \quad\quad\quad | \\ \quad\quad\quad Y-B-R_2 \end{array} \quad (I),$$

in welcher

- $R_1$ einen Aryl-, Aryloxy-, Arylthio-, Arylsulfinyl-, Arylsulfonyl- oder Arylaminorest mit 6-14 Kohlenstoffatomen, wobei die Arylteile durch einen oder mehrere Reste aus der Gruppe Hydroxy, Halogen, $C_1$-$C_8$-Alkyl, $C_1$-$C_8$-Alkoxy, Trifluormethyl, Cyano, Nitro, Amino, $C_1$-$C_8$-Alkylamino oder Di-$C_1$-$C_8$-Alkylamino substituiert sein können,
- $R_2$ einen Arylrest mit 6 bis 14 Kohlenstoffatomen, der durch einen oder mehrere Reste aus der Gruppe Hydroxy, Halogen, $C_1$-$C_8$-Alkyl, $C_1$-$C_8$-Alkoxy, Trifluormethyl, Cyano, Nitro, Amino, $C_1$-$C_8$-Alkylamino oder Di-$C_1$-$C_8$-Alkylamino substituiert sein kann,
- A einen geradkettigen oder verzweigten, gesättigten oder ungesättigten aliphatischen Rest mit 3-10 Kohlenstoffatomen, der gegebenenfalls durch ein Sauerstoffatom oder die Gruppe $S(O)_n$ mit n = O, 1 oder 2 oder -NH- unterbrochen ist und eine Kettenlänge von mindestens 3 C-Atomen besitzt, mit der Maßgabe, daß an einem ungesättigten aliphatischen Kohlenstoffatom kein Heteroatom stehen soll,
- Y die Gruppen -$S(O)_n$- oder -O-,
- n die Zahlen 0, 1 oder 2 und
- B einen Valenzstrich oder einen geradkettigen oder verzweigten, gesättigten oder ungesättigten aliphatischen Rest mit 1 bis 5 Kohlenstoffatomen

bedeutet, sowie deren physiologisch unbedenklichen Salze und Ester mit 1-8 C-Atomen.

**2.** R- und S-Carbonsäuren der Formel I gemäß Anspruch 1, dadurch gekennzeichnet, daß $R_1$ einen Phenyl-, Phenoxy-, Phenylthio-, Phenylsulfinyl-, Phenylsulfonyl- oder Phenylaminorest bedeutet, der gegebenenfalls ein- oder mehrfach durch Halogen, Hydroxy-, $C_1$-$C_8$-Alkyl-, $C_1$-$C_8$-Alkoxy- und Trifluormethylgruppen substituiert sein kann, insbesondere der 4-Chlorphenyl- oder 2-Methoxyphenylrest.

**3.** R- und S-Carbonsäuren der Formel I gemäß Anspruch 1 oder 2, dadurch gekennzeichnet, daß $R_2$ einen Phenylrest bedeutet, der gegebenenfalls ein- oder mehrfach durch Halogen, Hydroxy-, $C_1$-$C_8$-Alkyl-, $C_1$-$C_8$-Alkoxy- und Trifluormethylgruppen substituiert sein kann, insbesondere der 4-Methylphenyl- oder 4-tert.-Butylphenylrest.

**4.** R- und S-Carbonsäuren der Formel I gemäß Anspruch 1, 2 oder 3, dadurch gekennzeichnet, daß A die Gruppen $-(CH_2)_o-$ mit o = 3-10 oder $-(CH_2)_p-X-(CH_2)_q$ mit p = 2-8 und q = 1-6 bedeutet, wobei o, p, q ganze Zahlen bedeuten und die Summe von p und q nicht größer als 10 sein darf und X ein Sauerstoffatom bedeutet, oder, falls $R_1$ einen wie in Definition von $R_1$ angegebenen Phenylrest darstellt, A auch die Gruppen $-CH_2-X-(CH_2)_q-$, $-CH=CH-CH_2-$, $-C\equiv C-CH_2-$, $-CH=CH-(CH_2)_p-$, $-C\equiv C-(CH_2)_p-$ darstellt, wobei p, q und X die oben angegebene Definition besitzen.

**5.** R- und S-Carbonsäuren der Formel I gemäß einem der Ansprüche 1-4, dadurch gekennzeichnet, daß B einen Valenzstrich oder die Gruppe $-(CH_2)_r-$ mit r = 1-5, $-CH_2-CH=CH-$, $-CH_2-C\equiv C-$ oder $-CH=CH-$ bedeutet.

**6.** R- und S-Carbonsäuren der Formel I gemäß einem der Ansprüche 1-5, dadurch gekennzeichnet, daS Y ein Sauerstoffatom bedeutet.

**7.** R- und S-Carbonsäuren der Formel I gemäß einem der Ansprüche 1-6, dadurch gekennzeichnet, daß Y ein Sauerstoffatom und B einen Valenzstrich bedeutet.

**8.** Carbonsäurederivate der Formel I gemäß Anspruch 1 ausgewählt aus der Gruppe der folgenden Verbindungen:

(-)-7-(4-Chlorphenyl)-2-(4-methylphenylthio)heptansäure

(-)-5-(4-Chlorphenyl)-2-(4-methylphenylsulfonyl)-pentinsäure

(-)-7-(4-Chlorphenyl)-2-(4-methylphenoxy)heptansäure

(-)-6-(4-Chlorphenoxy)-2-(4-methylphenoxy)hexansäure

(-)-7-(4-Chlorphenyl)-2-[4-(1,1-dimethylethyl)phenoxy]heptansäure

(-)-2-[4-(1,1-Dimethylethyl)phenoxy]-6-phenoxyhexansäure

(-)-7-(2-Methoxyphenyl)-2-[4-(1,1-dimethylethyl)phenoxy]heptansäure

sowie deren physiologisch verträgliche Salze oder $C_1$-$C_6$-Alkylester.

**9.** Verfahren zur Herstellung von R- und S-Carbonsäuren der Formel I gemäß einem der Ansprüche 1-8, dadurch gekennzeichnet, daß man

a) in an sich bekannter Weise ein racemisches Gemisch von Verbindungen der allgemeinen Formel I mit optisch aktiven Basen umsetzt und die entstandenen diastereomeren Salze durch physikalische Methoden, wie z.B. fraktionierte Kristallisation oder Fest-Flüssigchromatographie, trennt und die Säuren wieder freisetzt,

b) in an sich bekannter Weise ein racemisches Gemisch eines niederen Esters von Verbindungen der allgemeinen Formel I mit einem Enzym wie Esterase oder Lipase enantioselektiv spaltet oder

c) in an sich bekannter Weise eine optisch aktive Verbindung der allgemeinen Formel II

$$R_1-A-\overset{*}{C}H(X)-COOR_3 \qquad (II),$$

S-Isomer oder R-Isomer

in welcher $R_1$ und A die oben angegebenen Bezeichnungen besitzen und X eine Abgangsgruppe wie z.B. Halogen oder einen Sulfonsäureester und $R_3$ einen niederen $C_1$-$C_8$-Alkylrest bedeuten,

mit einer Verbindung der allgemeinen Formel III

$H-Y-B-R_2$ (III),

in welcher B und $R_2$ die oben angegebenen Bedeutungen besitzen und Y S oder O bedeutet, umsetzt und die erhaltenen Ester der Verbindungen der allgemeinen Formel I im Anschluß daran durch Verseifen in die freien Carbonsäuren der allgemeinen Formel I überführt

und gegebenenfalls die Derivate mit Y = S in an sich bekannter Weise durch Oxidation des Schwefels in Derivate mit Y = SO bzw. $SO_2$ überführt.

**10.** Arzneimittel enthaltend mindestens eine R- oder S-Carbonsäure der Formel I gemäß einem der Ansprüche 1-8, sowie pharmazeutisch übliche Träger- oder Zusatzstoffe.

**11.** Verwendung von R- und S-Carbonsäuren der Formel I gemäß einem der Ansprüche 1-8 zur Herstellung von Arzneimitteln mit blutzuckersenkender Wirkung.

**12.** Verwendung von R- und S-Carbonsäuren der Formel I gemäß einem der Ansprüche 1-8 zur Herstellung von Arzneimitteln zur Behandlung von Diabetes, Prädiabetes, Altersdiabetes oder Fettstoffwechselerkrankungen, insbesondere von Atherosklerose oder Adipositas.

Europäisches
Patentamt

# EUROPÄISCHER RECHERCHENBERICHT

Nummer der Anmeldung

EP 92 10 5476

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,,, X | EP-A-0 279 162 (BOEHRINGER MANNHEIM GMBH)<br>* Seite 4, Zeile 55 - Zeile 57 *<br>* Seite 9, Zeile 6 *<br>* Seite 10, Zeile 36; Ansprüche 1,7-10 *<br>--- | 1,8, 10-12 | C07C59/68 |
| Y | DE-B-2 122 273 (WILLIAM H. RORER INC.)<br>* Spalte 6, Zeile 21 - Zeile 41; Ansprüche 1,3 *<br>--- | 1,9-12 | |
| Y | DE-A-2 651 500 (ALBERT ROLLAND S.A.)<br>* Seite 7, Zeile 27 - Seite 8, Zeile 3; Ansprüche 1,2,8 *<br>----- | 1,9-12 | |

RECHERCHIERTE SACHGEBIETE (Int. Cl.5 )

C07C

Der vorliegende Recherchenbericht wurde für alle Patentansprüche erstellt

| Recherchenort | Abschlußdatum der Recherche | Prüfer |
|---|---|---|
| DEN HAAG | 15 JUNI 1992 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument